(19) **Europäisches Patentamt**

**European Patent Office**

**Office européen des brevets**

(11) Publication number: **0 296 751 B1**

(12) **EUROPEAN PATENT SPECIFICATION**

(45) Date of publication of patent specification: **14.10.92**  (51) Int. Cl.5: **A61K 31/20**

(21) Application number: **88305450.4**

(22) Date of filing: **15.06.88**

(54) **Essential fatty acid compositions.**

(30) Priority: **24.06.87 GB 8714772**

(43) Date of publication of application:
**28.12.88 Bulletin 88/52**

(45) Publication of the grant of the patent:
**14.10.92 Bulletin 92/42**

(84) Designated Contracting States:
**AT BE CH DE ES FR GB GR IT LI LU NL SE**

(56) References cited:
**EP-A- 0 115 419**
**EP-A- 0 234 733**
**EP-A- 0 275 643**

**CHEMICAL ABSTRACTS, vol. 106, no. 23, 08
June 1987, Columbus, OH (US);
N.YAMAMOTO et al., p. 639, no.
195209n&NUM;**

**CHEMICAL ABSTRACTS, vol. 101, no. 9, 27
August 1984, Columbus, OH (US);
H.L.RUETHRICH et al., p. 631, no.
71570a&NUM;**

(73) Proprietor: **EFAMOL HOLDINGS PLC
Efamol House Woodbridge Meadows
Guildford Surrey GU1 1BA(GB)**

(72) Inventor: **Horrobin, David Frederick
c/o Efamol House Woodbridge Meadows
Guildford Surrey GU1 1BA(GB)**

(74) Representative: **Miller, Joseph et al
J. MILLER & CO. 34 Bedford Row, Holborn
London WC1R 4JH(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The invention relates to essential fatty acid (EFA) compositions and treatments therewith.

A previously filed but not prior published patent application EP-A-0,234,733 has dealt with the use of lithium and/or essential fatty acids (EFAs) in the treatment of dementias, including Alzheimer's disease. Memory loss can occur in the dementias, but also in many other situations such as alcoholism, chronic schizophrenia and normal ageing. We have now found that EFA supplementation as such is beneficial in memory loss.

**GENERAL DISCUSSION**

The bodily EFAs, falling largely into the two series known as the n-6 and n-3 EFAs of structure and relation as follows, and sharing, it is believed, common enzymes in the two pathways, are:

```
    n-6                                  n-3
18:2 delta-9,12(linoleic acid)       18:3 delta-9,12,15
                                     (alpha-linolenic acid)
        delta-6 desaturase ↓
18:3 delta-6,9,12(gamma-linolenic   18:4 delta-6,9,12,15
                           acid)
            elongation ↓
20:3 delta-8,11,14(dihomo-gamma-    20:4 delta-8,11,14,17
            linolenic acid)
        delta-5 desaturase ↓
20:4 delta-5,8,11,14(arachidonic    20:5 delta-5,8,11,14,17
                          acid)
            elongation ↓
22:4 delta-7,10,13,16(adrenic       22:5 delta-7,10,13,16,19
                        acid)
        delta-4 desaturase ↓
22:5 delta-4,7,10,13,16             22:6 delta-4,7,10,13,16,19
```

The acids are in the natural all-cis configuration. In the n-6 series, commonly used names for the 18:2 and 18:3 (octadeca di-enoic and tri-enoic) acids are linoleic acid and gamma-linolenic acid (GLA); for the 20:3 and 20:4 (eicosa tri-enoic and tetra-enoic) acids they are dihomo-gamma-linolenic acid (DGLA) and arachidonic acid (AA); and for the 22:4 (docosatetraenoic) acid adrenic acid. In the n-3 series only alpha-linolenic acid (ALA) (18:3) is commonly referred to by a non-systematic name but the name stearidonic acid does exist for the 18:4 n-3 acid. Initials derived from the systematic names are also used e.g. EPA for the 20:5 (eicosapentaenoic) acid but do not serve where acids of the same chain length and degree of unsaturation exist in both series e.g. the two 22:5 acids.

**BACKGROUND OF THE INVENTION**

The brain is particularly rich in EFAs, but the parent dietary EFAs, namely linoleic acid and alpha-linolenic acid, are present only in small amounts. These dietary EFAs must be metabolised by the enzyme delta-6-desaturase (D6D), to give (from linoleic acid) gamma-linolenic acid (GLA) and (from alpha-linolenic acid) stearidonic acid 18:4 n-3, and then to other metabolites. Because ageing, alcohol, catecholamines released during stress, and lack of certain nutritional factors have all been shown to impair D6D function, it occurred to us to see if there was a particular value for the brain in administering GLA, 18:4 n-3 and the higher acids formed from them.

**EXPERIMENTAL WORK**

Human studies have now been performed in two groups of patients known to suffer from memory impairment. These are alcoholics and chronic hospitalised schizophrenics. In alcoholics withdrawing from alcohol, either evening primrose oil capsules or matching placebos were administered for a period of 24 weeks. At the start and end of this period, standard Stirling memory tests were administered. Evening primrose oil was used because it is unusual among vegetable oils in containing GLA as well as linoleic acid. The patients receiving evening primrose oil improved to a greater extent than those receiving placebo.

**Table 1 -**

Improvements in short term and long term memory on Stirling visual memory testing in groups of matched alcoholics given either evening primrose oil or placebo for 24 weeks. Each figure shows the percentage of incorrect answers ± the standard deviation. In the change column + indicates improvement and - deterioration.

### Short Term Memory % Incorrect

|  | GLA GROUP (n=14) | PLACEBO GROUP (n=14) |
|---|---|---|
| Start | 16.67 ± 13.20 | 14.55 ± 7.89 |
| End | 11.67 ± 11.74 | 16.82 ± 14.54 |
| Change | + 5.00 | − 2.27 |

### Long Term Memory % Incorrect

|  | GLA GROUP (n=14) | PLACEBO GROUP (n=14) |
|---|---|---|
| Start | 60.0 ± 15.37 | 55.0 ± 14.14 |
| End | 50.0 ± 21.0 | 56.82 ± 11.02 |
| Change | + 10.0 | − 1.82 |

Since linoleic acid but not GLA is present in the normal diet in substantial amounts, it is reasonable to assume that the GLA was responsible for this effect. The GLA metabolites, dihomo-gamma-linolenic acid (DGLA), arachidonic acid (AA), adrenic acid (AdrA) and 22:5 n-6 are also all present in human brain and will have similar therapeutic effects.

In a similar study in chronic hospitalised schizophrenics, GLA in the form of evening primrose oil also improved memory to a significantly greater degree than placebo, this time using the standard Wechsler battery of memory tests (Table 2, 1st phase).

In the schizophrenics at the end of the first trial, a second phase trial was carried out. The purpose was to test the possible contribution of the n-3 EFAs formed from alpha-linolenic acid. Convenient sources of these include 18:4n-3 in blackcurrant seed oil and eicosapentaenoic acid (20:5n-3) and docosahexaenoic acid (22:6n-3) from fish oils. A combination of both n-6 and n-3 EFAs was given to both groups of schizophrenics, those who had been on placebo and those who had been on active treatment. The improvement in memory as assessed by the Wechsler scale was greater in the patients treated with both n-3 and n-6 EFAs than in those treated with n-6 EFAs alone (Table 2, 2nd phase).

**Table 2 -**

Mean changes in Wechsler Memory Quotient in groups of schizophrenics given either evening primrose oil (containing n-6 EFAs) or placebo for a period of 16 weeks (first treatment period). The figures show the change in score from baseline with a + sign indicating improvement. In a second phase of the study, both groups were given a preparation containing both n-6 and n-3 EFAs for a further 16 weeks. Further improvements were noted and again the figures show the changes from the original baseline.

|                    | 1st Phase | 2nd Phase |
|--------------------|-----------|-----------|
| Placebo (n=19)     | +0.9      | +7.6      |
| Primrose oil (n=12)| +6.1      | +9.3      |

## THE INVENTION

The invention, therefore, broadly lies in:

1. A method for the manufacture of a medicament for treatment or prevention of memory loss other than that due to senile or pre-senile dementia, of an n-6 essential fatty acid selected from the 18:3, 20:3, 20:4, 22:4 and 22:5 n-6 acids and an n-3 essential fatty acid selected from the 18:4, 20:4, 20:5, 22:5 and 22:6 n-3 acids.

2. Preparation of medicaments for use in improving memory wherein n-6 and n-3 EFAs are associated in amounts suited to said doses.

## FORMS AND SOURCES OF EFAs

When derivatives of the EFAs are used as discussed below, the amounts are calculated as the EFA.

The EFAs can be and indeed normally will be used as an assimilable, pharmacologically acceptable and physiologically equivalent derivative and reference to EFAs herein, including in the claims, is to be taken as including reference to such derivatives. Identification of useful derivatives is by their having the valuable effect in the body of the acid itself, but conversion can be shown directly by gas chromatographic analysis of concentrations in blood, body fat, or other tissue by standard techniques, for example those of Pelick et al, p. 23, "Analysis of Lipids and Lipoproteins" Ed. Perkins, American Oil Chemists Society, Champaign, Illinois, U.S.A.

Convenient derivatives of EFAs include salts, amides, esters including glyceride esters and alkyl (e.g. $C_1$ to $C_4$) esters, and phospholipids.

Thus if desired, pharmaceutical compositions may be produced for use in the invention so far as the EFA is concerned by associating the natural or synthetic acid, as such or as a derivative, with an acceptable pharmaceutical vehicle. It is, however, at present convenient to incorporate the acids into compositions in the form of available oils having a high content of the acids, hence references to "oils" herein.

Fish oils are a convenient source of n-3 EFAs, examples being fish body oils, especially of oily fish such as herring, mackerel, anchovies, pilchards, menhaden, tuna and salmon, fish gut oils, fish liver oils; oils derived from growth of certain fungi and algae; oils from animals or birds consuming fish, such as seals.

At the present time known natural oils having a high GLA acid content are few (there are no known natural sources of significant amounts of DGLA). One source of GLA currently available is the seed of Evening Primrose species such as Oenothera biennis L. and Oenothera lamarckiana, the oil extract therefrom containing GLA (about 8%) and linoleic acid (about 72%) in the form of their glycerides together with other glycerides (percentages based on total fatty acids). Other sources of GLA are Borage species such as Borago officinalis which, though current yield per acre is low, provide a richer source of gamma-linolenic acid than Oenothera oil. Recent studies on fungi and other micro-organisms which can be cultivated by fermentation promise a micro-organism source.

The oil is extracted from the seeds by one of the conventional methods of extraction such as cold pressure, screw pressure after partially cooking the seed or solvent extraction.

Fractionation of a typical sample of this oil in the form of methyl esters shows the relative proportions:

| Palmitate | 6.15 |
| Stearate | 1.6 |
| Oleate | 10.15 |
| Linoleate | 72.6 |
| Gamma-Linolenate | 8.9 |

The seed oil extracts referred to above can be used as such or can, for example, if desired, be fractionated to yield an oily composition containing the triglycerides of gamma-linolenic and linoleic as the main fatty acid components, the gamma-linolenic acid content being if desired a major proportion. Seed oil extracts appear to have a stabilising effect upon DGLA if present.

For the n-6 series acids higher than GLA synthesis is possible though not simple, for example for DGLA. However AA and higher acids are available from slaughterhouses, for example adrenic acid from adrenal glands. Arachidonic acid is present in substantial amounts in meat.

## PACKS

If it is not desired to have compositions comprising different active materials together, packs may be prepared comprising the materials presented for separate, or part joint and part separate administration in the appropriate relative amounts, and use of such packs is within the purview of this invention.

## DIETARY COMPOSITIONS

The invention is chiefly described in relation to pharmaceutical compositions, but it will be understood that the EFAs, being in the nature of dietary supplements, could be incorporated in a dietary margarine or other foodstuff which when for the purposes herein is to be considered a medicament.

## PHARMACEUTICAL PRESENTATION

The compositions are conveniently in a form suitable for oral, parenteral, administration in a suitable pharmaceutical vehicle, as discussed in detail for example in Williams GB-A-1,082,624 to which reference may be made, and in any case very well known generally for any particular kind of preparation. Thus, for example, tablets, capsules, ingestible liquid or powder preparations can be prepared as required. Injectable solutions of hydrolysed Oenothera oil and fish oil may be prepared using albumin to solubilise the free acid.

It will be understood that the absolute quantity of active materials present in any dosage unit should not exceed that appropriate to the rate and manner of administration to be employed but on the other hand should also desirably be adequate to allow the desired rate of administration to be achieved by a small number of doses. The rate of administration will moreover depend on the precise pharmacological action desired.

## EXAMPLES

The following are specific examples of the use of essential fatty acids in medicaments, for use in treatment or prophylaxis in man against memory loss:-

1. 500 mg capsules of blackcurrant seed oil containing 90 mg GLA and 20 mg of 18:4n-3, six per day.

2. 500 mg capsules containing 300 mg ethyl-GLA and 200 mg ethyl-EPA, six per day.

3. 500 mg capsules containing 80% evening primrose oil (9% GLA and 72% linoleic acid) and 20% fish oil (18% EPA and 12% DHA), twelve per day.

## Claims

## Claims for the following Contracting States : BE, CH, DE, FR, GB, IT, LI, LU, NL, SE

1. Use, for the manufacture of a medicament for treatment or prevention of memory loss other than that due to senile or pre-senile dementia, of an n-6 essential fatty acid selected from the 18:3, 20:3, 20:4, 22:4 and 22:5 n-6 acids and an n-3 essential fatty acid selected from the 18:4, 20:4, 20:5, 22:5 and 22:6 n-3 acids.

2. The use claimed in claim 1, wherein said medicament is presented for administration of from 1 mg to 100 g of the n-6 acid and of the n-3 acid daily.

3. The use claimed in claim 1, wherein said medicament is presented for administration of from 10 mg to 10 g of the n-6 acid and of the n-3 acid daily.

**Claims for the following Contracting States : AT, ES**

1. Method for the manufacture of a medicament for treatment of prevention of memory loss other than that due to senile or pre-senile dementia, charactersied in that an n-6 essential fatty acid selected from the 18:3, 20:3, 20:4, 22:4 and 22:5 n-6 acids and an n-3 essential fatty acid selected from the 18:4, 20:4, 20:5, 22:5 and 22:6 n-3 acids are used.

2. Method according to Claim 1, wherein said medicament is prepared for administration of from 1 mg to 100 g of the n-6 acid and of the n-3 acid daily.

3. Method according to Claim 1, wherein said medicament is prepared for administration of from 10 mg to 10g of the n-6 acid and of the n-3 acid daily.

4. Method according to any of Claims 1 to 3, wherein said medicament is prepared by combining 500 mg blackcurrent seed oil containing 90 mg GLA and 20 mg of 18:4 n-3; or 300 mg ethyl-GLA and 200 mg ethyl-EPA; or 80% evening primrose oil and 20% fish oil, into a 500 mg capsule.

**Claim for the following Contracting State : GR**

1. Use, for the manufacture of a composition for treatment or prevention of memory loss other than that due to senile or pre-senile dementia, of an n-6 essential fatty acid selected from the 18:3, 20:3, 20:4, 22:4 and 22:5 n-6 acids and an n-3 essential fatty acid selected from the 18:4, 20:4, 20:5, 22:5 and 22:6 n-3 acids.

**Patentansprüche**
**Patentansprüche für folgende Vertragsstaaten : BE, CH, DE, FR, GB, IT, LI, LU, NL, SE**

1. Verwendung, für die Herstellung eines Medikamentes zur Behandlung oder Vorbeugung von Gedächtnisverlust, und zwar einem anderen als dem, der von seniler oder prä-seniler Demenz kommt, einer n-6 essentiellen Fettsäure, die aus den 18:3, 20:3, 20:4, 22:4 und 22:5 n-6 Säuren ausgewählt wird und einer n-3 essentiellen Fettsäure, die aus den 18:4, 20:4, 20:5, 22:5 und 22:6 n-3 Säuren ausgewählt wird.

2. Verwendung gemäß Anspruch 1, wobei besagtes Medikament zur Verabreichung von 1 mg bis 100 g der n-6 Säure und der n-3 Säure täglich präsentiert wird.

3. Verwendung gemäß Anspruch 1, worin besagtes Medikament zur Verabreichung von 10 mg bis 10 g der n-6 Säure und der n-3 Säure täglich präsentiert wird.

**Patentansprüche für folgende Vertragsstaaten : AT, ES**

1. Verfahren für die Herstellung eines Medikamentes für die Behandlung oder Vorbeugung von Gedächtnisverlust, und zwar einem anderen als dem, der von seniler oder prä-seniler Demenz kommt, dadurch gekennzeichnet, daß eine n-6 essentielle Fettsäure, die aus den 18:3, 20:3, 20:4, 22:4 und 22:5 n-6 Säuren ausgewählt wird und eine n-3 essentielle Fettsäure, die aus den 18:4, 20:4, 20:5, 22:5 und 22:6 n-3 Säuren ausgewählt wird, verwendet werden.

2. Verfahren gemäß Anspruch 1, worin besagtes Medikament zubereitet wird zur täglichen Verabreichung von 1 mg bis 100 g der n-6 Säure und von n-3 Säure.

3. Verfahren gemäß Anspruch 1, worin besagtes Medikament zubereitet wird zur täglichen Verabreichung von 10 mg bis 10 g der n-6 Säure und von der n-3 Säure.

EP 0 296 751 B1

4. Verfahren gemäß einem der Ansprüche 1 bis 3, worin besagtes Medikament zubereitet wird durch Kombinieren von 500 mg Samen der schwarzen Johannisbeere, das 90 mg GLA enthält und 20 mg der 18:4 n-3; oder 300 mg Ethyl-GLA und 200 mg Ethyl-EPA; oder 80 % Nachtkerzenöl und 20 % Fischöl in eine 500 mg Kapsel.

**Patentanspruch für folgenden Vertragsstaat : GR**

1. Verwendung, für die Herstellung einer Zusammensetzung für die Behandlung oder Vorbeugung von Gedächtnisverlust, und zwar einem anderen als dem, der von seniler oder prä-seniler Demenz kommt, einer n-6 essentiellen Fettsäure, die aus den 18-3, 20:3, 20:4, 22:4 und 22:5 n-6 Säuren ausgewählt wird und einer n-3 essentiellen Fettsäure, die aus den 18:4, 20:4, 20:5, 22:5 und 22:6 n-3 Säuren ausgewählt wird.

**Revendications**

**Revendications pour les Etats contractants suivants : BE, CH, DE, FR, GB, IT, LI, LU, NL, SE**

1. Utilisation, pour la fabrication d'un médicament pour le traitement ou la prévention des pertes de mémoire autres que celles dues à la démence sénile ou pré-sénile, d'un acide gras essentiel n-6, choisi parmi les acides n-6 18:3, 20:3, 20:4, 22:4 et 22:5, et d'un acide gras essentiel n-3 choisi parmi les acides n-3 18:4, 20:4, 20:5, 22:5 et 22:6.

2. Utilisation selon la revendication 1, dans laquelle ledit médicament est présenté pour l'administration de 1 mg à 100 g de l'acide n-6 et de l'acide n-3 quotidiennement.

3. Utilisation selon la revendication 1, dans laquelle ledit médicament est présenté pour l'administration de 10 mg à 10 g de l'acide n-6 et de l'acide n-3 quotidiennement.

**Revendications pour les Etats contractants suivants : AT, ES**

1. Procédé de fabrication d'un médicament pour le traitement ou la prévention des pertes de mémoire autres que celles dues à la démence sénile ou pré-sénile, caractérisé par le fait qu'on utilise un acide gras essentiel n-6 choisi parmi les acides n-6 18:3, 20:3, 20:4, 22:4 et 22:5 et un acide gras essentiel n-3 choisi parmi les acides n-3 18:4, 20:4, 20:5, 22:5 et 22:6.

2. Procédé selon la revendication 1, dans lequel ledit médicament est préparé pour l'administration de 1 mg à 100 g de l'acide n-6 et de l'acide n-3 quotidiennement.

3. Procédé selon la revendication 1, dans lequel ledit médicament est préparé pour l'administration de 10 mg à 10 g de l'acide n-6 et de l'acide n-3 quotidiennement.

4. Procédé selon l'une quelconque des revendications 1 à 3, dans lequel ledit médicament est préparé par combinaison de 500 mg d'huile de pépins de cassis contenant 90 mg de GLA et 20 mg de n-3 18:4 ; ou 300 mg d'éthyl-GLA et 200 mg d'éthyl-EPA ; ou 80 d'huile d'oenothère et 20% d'huile de poisson, dans une capsule de 500 mg.

**Revendication pour l'Etat contractant suivant : GR**

1. Utilisation, pour la fabrication d'une composition pour le traitement ou la prévention des pertes de mémoire autres que celles dues à la démence sénile ou pré-sénile, d'un acide gras essentiel n-6 choisi parmi les acides n-6 18:3, 20:3, 20:4, 22:4 et 22:5, et d'un acide gras essentiel n-3 choisi parmi les acides n-3 18:4, 20:4, 20:5, 22:5 et 22:6.

7